# EUROPÄISCHE PATENTSCHRIFT

(11) **EP 2 409 725 B1**
(45) Veröffentlichungstag und Bekanntmachung des Hinweises auf die Patenterteilung: **08.10.2014**
(21) Anmeldenummer: 11172365.6
(22) Anmeldetag: 01.07.2011
(51) Int. Cl.: A61N 1/05, A61L 29/12

(54) **Isolationsschlauch für eine Elektrodenleitung zur medizinischen Anwendung**
Insulating tube for electrode lead for medical use
Tuyau d'isolation pour ligne d'électrodes pour application médicale

(30) Priorität: 22.07.2010 US 366529 P
(43) Veröffentlichungstag der Anmeldung: 25.01.2012
(73) Patentinhaber: BIOTRONIK SE & Co. KG, 12359 Berlin (DE)
(72) Erfinder: Günther, Thomas, 14552 Michendorf OT Wilhelmshorst (DE); Kolberg, Gernot, 12043 Berlin (DE)
(74) Vertreter: Lindner-Vogt, Karin L.

(56) Entgegenhaltungen:
- US-A- 6 078 839
- US-A1- 2005 137 664
- US-A1- 2009 210 043
- US-A1- 2011 112 614

## Beschreibung

Die vorliegende Erfindung betrifft einen Isolationsschlauch für eine Elektrodenleitung zur medizinischen Anwendung, insbesondere Herzschrittmacherelektrodenleitung, Defibrillatorelektrodenleitung, Elektrodenleitung zur Nervenstimulation oder dergleichen, enthaltend einen Grundwerkstoff, z.B. Silikon.

Elektronische medizinische Einrichtungen wie Herzschrittmacher (Pacemaker), Defibrillatoren oder neurologische Geräte wie Hirnschrittmacher für tiefe Hirnstimulation (Deep Brain Stimulation), Rückenmarkstimulationsgeräte, TENS (Transcutaneous Electrical Nerve Stimulator) oder Geräte für muskuläre Stimulationstherapie sowie Diagnosegeräte, welche die chemischen Eigenschaften des Blutes des Patienten, anderer Körperteile oder andere Körpereigenschaften und -parameter untersuchen, verwenden häufig Elektrodenleitungen, die in den Körper des Patienten geführt werden und dort zumindest über den Zeitraum der Behandlung oder Messung verbleiben. Die Elektrodenleitungen sind mit der ggf. implantierbaren elektronischen Einrichtung elektrisch leitend verbunden.

Diese medizinischen Einrichtungen umfassen üblicherweise ein ggf. körperverträgliches Gehäuse mit einer dazugehörigen elektronischen Schaltung und einer Energieversorgung, z.B. einer Batterie. Das Gehäuse besitzt mindestens eine Anschlussstelle, an der die Elektrodenleitung oder die Elektrodenleitungen angeschlossen werden können. Die Elektrodenleitung oder die Elektrodenleitungen dienen der Übertragung der elektrischen Energie vom Gehäuse zu dem behandelten oder untersuchten Körperteil und umgekehrt.

Hierbei bezeichnet der Begriff "Elektrodenleitung" in der Medizintechnik nicht nur ein Element, mit dem nach physikalischer Definition elektrische Energie übertragen wird, sondern umfasst eine Leitung mit einem elektrischen Leiter zusammen mit seiner umhüllenden Isolation, die häufig als Isolationsschlauch ausgebildet ist, sowie alle weiteren funktionellen Elemente, die mit der Leitung fest verbunden sind. Die Elektrodenleitung umfasst beispielsweise auch die sogenannte Elektrodenspitze, mittels der die elektrische Energie in das zu behandelnde Gewebe eingeleitet wird. Häufig ist eine Elektrodenspitze auch mit Ankerelementen oder Haltestrukturen versehen, mit denen die Konstanz der räumlichen Lage der Übergangsstelle der elektrischen Energie in dem zu behandelnden Gewebe sichergestellt wird. Die Elektrodenspitze, die eine Übergangsstelle der elektrischen Energie in das Gewebe bildet, kann als Ableit-, Stimulations- oder Messelektrode ausgelegt sein.

Bei derartigen Elektrodenleitungen wird häufig zur Isolation der elektrisch leitenden Elemente außerhalb des Anschlusses und der Elektrodenspitze ein Isolationsschlauch aus Silikon verwendet. Silikone weisen eine hohe Biokompatibilität, eine ausreichende Härte sowie sehr gute dauerelastische Eigenschaften auf.

Reibt eine Elektrodenleitung beispielsweise am Schlüsselbein oder an einer zweiten Elektrodenleitung (z.B. wenn mehrere Elektrodenleitungen implantiert sind), so kann es vorkommen, dass der Isolationsschlauch so weit durchgerieben wird, dass die Funktionsfähigkeit der Elektrodenleitung beeinträchtigt wird.

Aus diesem Grund wurde bereits nach Wegen gesucht, die Abriebfestigkeit eines Isolationsschlauchs zu erhöhen.

In der Druckschrift DE 10 2008 010 188 A1 wurde beispielsweise ein Isolationsschlauch vorgeschlagen, bei dem auf der Mantelfläche einer inneren, hohlzylinderförmigen Schicht eine zweite Schicht angeordnet ist, welche mindestens ein Polymer aus der Gruppe bestehend aus Polyurethan und Silikon-Polyurethan-Copolymer enthält. Polyurethan besitzt jedoch für viele Einsatzzwecke keine ausreichende Langzeitstabilität, da das Material mit der Zeit degradiert. Auch bei der vorgeschlagenen Zwei-Schicht-Lösung kann eine Degradation des Materials nicht grundsätzlich ausgeschlossen werden. Zudem ist eine solche Elektrodenleitung aufgrund der Anordnung von zwei Schichten übereinander deutlich härter als eine einschichtige Elektrodenleitung mit Silikonisolation. Dieser Effekt ist insbesondere für dünne Elektrodenleitungen relevant. Ferner ist das Konzept, einen Isolationsschlauch aus zwei koaxial angeordneten Schichten zu verwenden, fertigungstechnisch recht aufwendig.

In weiteren, bereits bekannten Lösungen wurden neue Werkstoffe entwickelt, welche die Eigenschaften von Silikon und Polyurethan verbinden, beispielsweise die Copolymere Pursil (Silikonpolyetherurethan), Carbosil (Polycarbonaturethan) oder Elast-Eon®. Diese Materialien sind noch so neu, dass für sie Langzeiterfahrungen fehlen. Demzufolge ist der Einsatz dieser Materialien für medizinische Anwendungen aufgrund umfangreicher notwendiger Tests ebenfalls aufwendig.

Aus der Druckschrift US 6078839 ist ein Schutzschlauch für eine implantierbare Elektrodenleitung bekannt, der eine verbesserte Abriebsfestigkeit durch eine eingebettete faserförmige Verstärkung erzielt.

Die Aufgabe der vorliegenden Erfindung besteht darin, einen Isolationsschlauch bzw. eine entsprechende Elektrodenleitung anzugeben, welche ein verbessertes Abriebverhalten sowie eine erhöhte Biegesteifigkeit aufweisen, ohne dass die Biokompatibilität oder die sonstigen mechanischen Eigenschaften wesentlich beeinträchtigt werden.

Die obige Aufgabe wird gelöst durch einen Isolationsschlauch enthaltend einen Grundwerkstoff aus mindestens einem Material der Gruppe enthaltend Silikone, Polyurethane, Polyimid, PTFE, ETFE, Copolymere aus Silikonen und Polyurethanen, wobei der Isolationsschlauch zumindest in einem vorzugsweise die Mantelfläche ausbildenden Volumenbereich zusätzlich zu dem Grundwerkstoff ein faserförmiges Füllmaterial aufweist, welches eine höhere Abriebfestigkeit als der jeweils verwendete Grundwerkstoff besitzt, und mindestens überwiegend derart ausgebildet ist, dass die Längsrichtung der Fasern parallel zu einer Längsachse des Isolationsschlauchs verläuft.

Der Vorteil des erfindungsgemäßen Isolationsschlauchs besteht darin, dass Grundwerkstoff nach wie vor die Eigenschaften des Isolationsschlauchs wie Biokompatibilität wesentlich bestimmt. Bei einem Angriff durch einen Reibepartner wird der Abrieb jedoch durch die in die Grundwerkstoff-Matrix eingebetteten abriebfesten Fasern gestoppt. Demzufolge kann bei einem erfindungsgemäßen Isolationsschlauch die Abriebfestigkeit insgesamt deutlich erhöht werden. Hinsichtlich der Eigenschaften Elastizität und Härte muss der Grundwerkstoff lediglich geringfügig angepasst werden, so dass das Gesamtmaterial aus Grundwerkstoff und Füllmaterial die gewünschte Gesamtelastizität und Gesamthärte auf weist.

Die vorliegende Erfindung beruht daher auf der Erkenntnis, dass nicht das gesamte Material des Isolationsschlauchs verändert werden muss, um die gewünschte Abriebfestigkeit sowie die gewünschte Biegesteifigkeit zu erreichen. Es ist vielmehr ausreichend, wenn lediglich ein geringer Anteil (Konzentrationsangaben siehe unten) des Materials durch ein abriebfesteres Füllmaterial ersetzt wird. Hierbei ist das faserförmige Füllmaterial hinsichtlich der Phase oder Phasenzusammensetzung verschieden von dem Grundwerkstoff. Dies bedeutet, dass in einer Faser bzw. einem Faserabschnitt ein räumlicher Bereich ausgebildet ist, in dem die bestimmenden physikalischen Parameter der Materie verschieden von den physikalischen Parametern der Materie des Grundwerkstoffs sind. Vorzugsweise besteht eine Faser bzw. ein Faserabschnitt aus einer einzigen Phase, d.h. einem hinsichtlich der physikalischen Parameter der Materie homogenen räumlichen Bereich.

In der vorliegenden Erfindung wird unter einem Silikon ein Poly(organo)siloxan, d.h. ein Polymer verstanden, bei dem Siliziumatome über Sauerstoffatome verknüpft sind. Ein solches Silikon kann Molekülketten und/oder -netze enthalten. Die restlichen freien Valenzelektronen des Siliziums sind dabei durch Kohlenwasserstoffreste abgesättigt. Für die vorliegende medizinische Anwendung eines Isolationsschlauchs werden insbesondere die folgenden Silikonkautschuke eingesetzt, vorzugsweise heißvernetzender Flüssigsilikonkautschuk (high temperature vulcanizing liquid silicone rubber oder kurz HTV LSR), der aus einer (zäh)flüssigen Phase extrudiert und bei hohen Temperaturen entweder peroxidisch induziert oder Platin-katalysiert vernetzt wird.

Vorteilhafte Werkstoffe, die das Füllmaterial bilden können, sind mindestens ein Werkstoff der Gruppe bestehend aus Kohlenstoff, Tantal, Platin, Gold, Iridium, Palladium, Silikonderivate, Copolymere aus Silikonen, keramisches Material, insbesondere Siliziumcarbid, Bariumtitanat, Titandioxid und Zirkoniumdioxid, und thermoplastisches Material, insbesondere Polyurethan, Polyamid, Polyethylen, Polyimid, PTFE (Polytetrafluorethylen), PFA (Perfluor-Alkoxyalkan), PET (Polyethylenterephthalat) und ETFE (Ethylen-Tetrafluorethylen).

Es soll an dieser Stelle ausdrücklich darauf hingewiesen werden, dass bei der Auswahl von Materialien aus den angegebenen Gruppen für den Grundwerkstoff und das Füllmaterial zur Realisierung der Erfindung stets Materialien mit unterschiedlichen Abriebfestigkeiten selektiert werden, wobei das Füllmaterial eine höhere Abriebfestigkeit besitzt als der Grundwerkstoff. Dies kann durch Auswahl unterschiedlicher Materialklassen oder durch Auswahl unterschiedlicher Varianten derselben, beispielsweise polymeren Materialklasse erfolgen. Bei der Verwendung unterschiedlicher Varianten ein und derselben Materialklasse für Grundwerkstoff und Füllmaterial kann unter Umständen eine sehr gute Verbundfestigkeit zwischen den Materialien von Grundwerkstoff und Füllmaterial erreicht werden. Beispielsweise kann das Füllmaterial aus bereits vernetzten Fasern aus abriebfesterem Silikon hergestellt werden. Dieses Füllmaterial kann dann der Extrusionsmasse aus überwiegend Silikon beigemengt werden, wobei sich das Füllmaterial, da es bereits vernetzt ist, dabei nicht auflöst.

Die Abriebfestigkeit kann insbesondere dann erhöht werden, wenn der Anteil des Füllmaterials an dem Material des Isolationsschlauchs 0,1 Vol.% bis 30 Vol.%, vorzugsweise 1 Vol.% bis 15 Vol.%, beträgt. Füllmaterial aus sehr kleinen Fasern (Nanomaterial) wird besonders bevorzugt in geringeren Konzentrationen im Bereich von 1 Vol.% bis 5 Vol.% zugefügt. Wie oben bereits erläutert wurde, ist, wie angegeben, bereits ein geringer Anteil an Füllmaterial mit einer höheren Abriebfestigkeit ausreichend, um die Eigenschaften des Isolationsschlauchs hinsichtlich des Abriebs signifikant zu verbessern, ohne die Eigenschaften wie Biokompatibilität des Matrix-Grundwerkstoffs zu beeinträchtigen. Bei diesen Konzentrationen wird bei der Verwendung von elektrisch leitendem Füllmaterial insgesamt auch die elektrische Isolierung der Elektrodenleitung durch den Isolationsschlauch ausreichend gewährleistet.

Aus gleichen Gründen beträgt in einem weiteren bevorzugten Ausführungsbeispiel die Länge des faserförmigen Füllmaterials 0,1 mm bis 10 mm. Hierbei wird die Länge des faserförmigen Füllmaterials insbesondere durch die gewünschte resultierende Steifigkeit des extrudierten oder gespritzten Schlauches bestimmt und stellt die Ausdehnung der Fasern in Längsrichtung dar. Insbesondere längere Fasern erhöhen in vorteilhafter Weise die Biegesteifigkeit des Isolationsschlauchs. In dieser Hinsicht ist es ebenfalls von Vorteil, wenn die Länge des faserförmigen Füllmaterials etwa das 10-fache bis 100-fache des Faserdurchmessers beträgt.

Besonders gute Ergebnisse zur Abriebfestigkeit werden erreicht, wenn das faserförmige Füllmaterial mindestens überwiegend derart ausgerichtet ist, dass die Längsrichtung der Fasern parallel zu einer Längsachse des Isolationsschlauchs verläuft. Hierbei wird unter Längsrichtung einer Faser die Richtung der jeweiligen Faser verstanden, entlang der die Faser die größte Ausdehnung besitzt. Die Längsausrichtung der Fasern führt zu einer axialen Verkrallung der resultierenden Schutzschicht. Bei einer Biegebeanspruchung des Schlauchmaterials wird so einer Kerbwirkung senkrecht zur Schlauchachse und damit einer Bruchgefahr entgegengewirkt. Radial angeordnete Fasern können die Biegelastwechselfestigkeit des Isolationsschlauches negativ beeinflussen.

Vorzugsweise ist das Füllmaterial über den Querschnitt des Isolationsschlauchs ungleichmäßig verteilt, beispielsweise lediglich in einer Schicht oder Teilschicht oder mit einem Gradienten. Bei ungleichmäßiger Verteilung des Füllmaterials werden gezielt die Bereiche des Isolationsschlauchs mit dem Füllmaterial versehen, die besonders gegen Abrieb geschützt werden sollen, beispielsweise die Bereiche in der Nähe der äußeren Mantelfläche des Isolationsschlauchs und/oder in der Nähe der Innenwand der Öffnung(en) in dem Isolationsschlauch, in der oder denen die Leiterelemente angeordnet sind.

Die obige Aufgabe wird mit den oben dargestellten Vorteilen auch durch eine Elektrodenleitung gelöst, welche einen Isolationsschlauch mit oben angegebenen Eigenschaften aufweist, wobei der Isolationsschlauch in einem Abschnitt der Elektrodenleitung, in dem dieser angeordnet ist, vorzugsweise die äußere Oberfläche der Elektrodenleitung ausbildet bzw. die Elektrode außen umgibt. Der Isolationsschlauch isoliert die inneren elektrisch leitenden Elemente der Elektrodenleitung nach außen und stellt zusätzlich einen Schutz gegen mechanische Reibung mit einem Reibepartner dar.

Weitere Ziele, Merkmale, Vorteile und Anwendungsmöglichkeiten der Erfindung ergeben sich aus der nachfolgenden Beschreibung und den Figuren, die sich jedoch auf Füllmaterialien aus Kugelförmigen Partikeln beziehen, und deshalb nicht Gegenstand der Erfindung sind.

Es zeigen schematisch:
- Fig. 1: einen Querschnitt eines ersten Biespiels eines Isolationsschlauchs,
- Fig. 2: ein Ausführungsbeispiel einer Elektrodenleitung in einer Ansicht von der Seite,
- Fig. 3: einen Querschnitt eines zweiten Biespiels eines Isolationsschlauchs,
- Fig. 4: einen Querschnitt eines dritten Beispiels eines Isolationsschlauchs,
- Fig. 5: einen Querschnitt eines vierten Beispiels eines Isolationsschlauchs,
- Fig. 6: einen Querschnitt eines fünften Beispiels eines Isolationsschlauchs,
- Fig. 7: einen Querschnitt eines sechsten Beispiels eines Isolationsschlauchs und
- Fig. 8: einen Querschnitt eines siebten Beispiels eines Isolationsschlauchs.

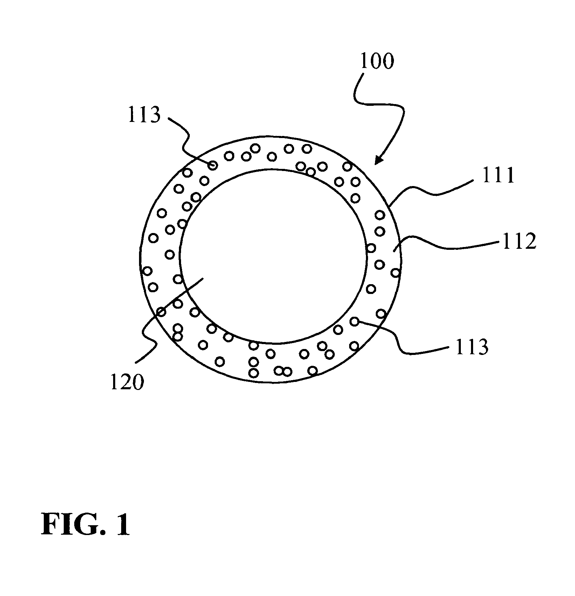

Der in Figur 1 dargestellte Isolationsschlauch 100 weist lediglich eine einzige Schicht 111 auf, welche im Wesentlichen hohlzylindrisch ausgebildet ist. Die Schicht 111 setzt sich zusammen aus einer Grundwerkstoff-Matrix 112 aus LSR (Liquid Silicone Rubber, Flüssigsilikon mit dem Hauptbestandteil lineare Siloxane), in die kugelförmige Partikel 113, beispielsweise aus Bariumtitanat, als Füllmaterial eingebettet sind. Derartige Bariumtitanat-Partikel 113 weisen einen mittleren Durchmesser von etwa 2 µm auf. Alternativ oder zusätzlich können auch Tantal-Partikel als Füllmaterial in die Grundwerkstoff-Matrix eingebettet werden. Diese haben einen mittleren Durchmesser von etwa 40 µm. Die durchgehende zylinderförmige Öffnung 120 des Isolationsschlauchs 100 dient der Aufnahme der leitenden Elemente, welche am distalen Ende der Elektrodenleitung die Elektrode oder die Elektroden und am proximalen Ende ein Verbindungselement zur elektrisch leitenden Verbindung mit einer elektronischen Einrichtung ausbilden. Die Bariumtitanat-Partikel und/oder die Tantal-Partikel 113 besitzen jeweils eine höhere Abriebfestigkeit als der Grundwerkstoff LSR 112.

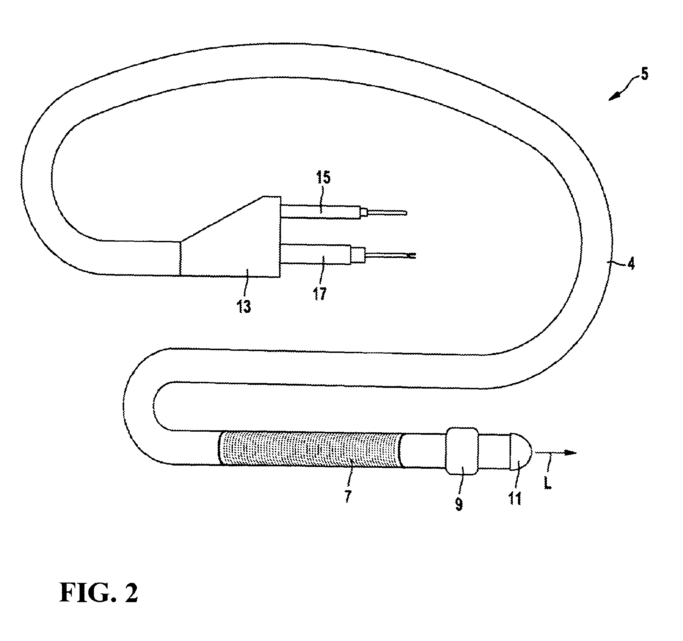

Figur 2 zeigt die Anwendung eines derartigen Isolationsschlauchs 100 am Beispiel einer implantierbaren Schockelektrodenleitung 5, welche in einem Defibrillator eingesetzt wird. In Figur 2 ist der Isolationsschlauch mit dem Bezugszeichen 4 versehen. Dieser kann analog zu dem in Figur 1 dargestellten Isolationsschlauch 100 oder analog zu einem der nachfolgend beschriebenen Beispiele aufgebaut sein. Die Elektrodenleitung 5 weist in Längsrichtung L ein proximales Ende und ein distales Ende auf. An dem distalen Ende besitzt die Schockelektrodenleitung 5 eine langgestreckte Elektrode 7, die z.B. in Wandkontakt im Herzen eines Patienten verlegt wird, sowie eine Ring-Elektrode 9 und eine Tip-Elektrode 11, welche für Abtast- und/oder Stimulationszwecke vorgesehen sind. Am proximalen Ende der Schockelektrodenleitung 5 sind aus einem Y-Verteiler 13 sich hinaus erstreckende Steckerelemente 15, 17 vorgesehen, die der Elektrode 7 und den Stimulations- bzw. Detektionselektroden 9,11 zugeordnet sind und zur elektrischen Verbindung mit einem (nicht dargestellten) implantierbaren Gerät dienen.

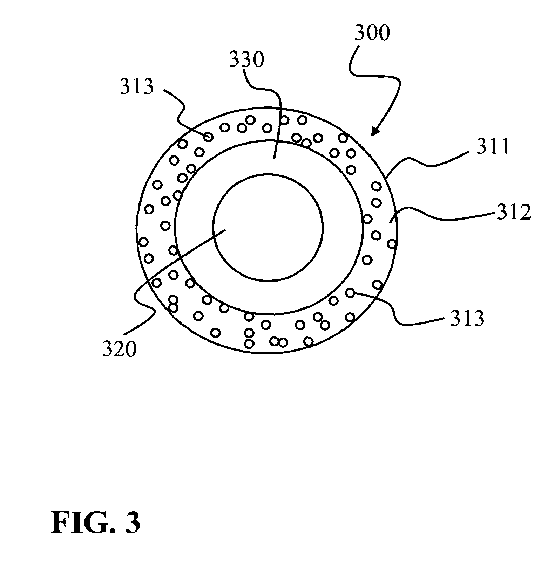

In dem in Figur 3 dargestellten zweiten Beispiel ist der Isolationsschlauch 300 aus zwei Schichten aufgebaut. Die innere Schicht 330, die im Wesentlichen hohlzylindrisch mit einer durchgehenden Öffnung 320 zur Aufnahme der leitenden Elemente geformt ist, besteht aus LSR. Konzentrisch zur inneren Schicht 320 ist eine zweite Schicht 311 auf der äußeren Oberfläche (Mantelfläche) der inneren Schicht 320 angeordnet, welche analog zu dem ersten Beispiel des Isolationsschlauchs eine LSR Grundwerkstoff-Matrix 312 und Partikel 313 aus Bariumtitanat mit einem mittleren Durchmesser von etwa 2 µm aufweist.

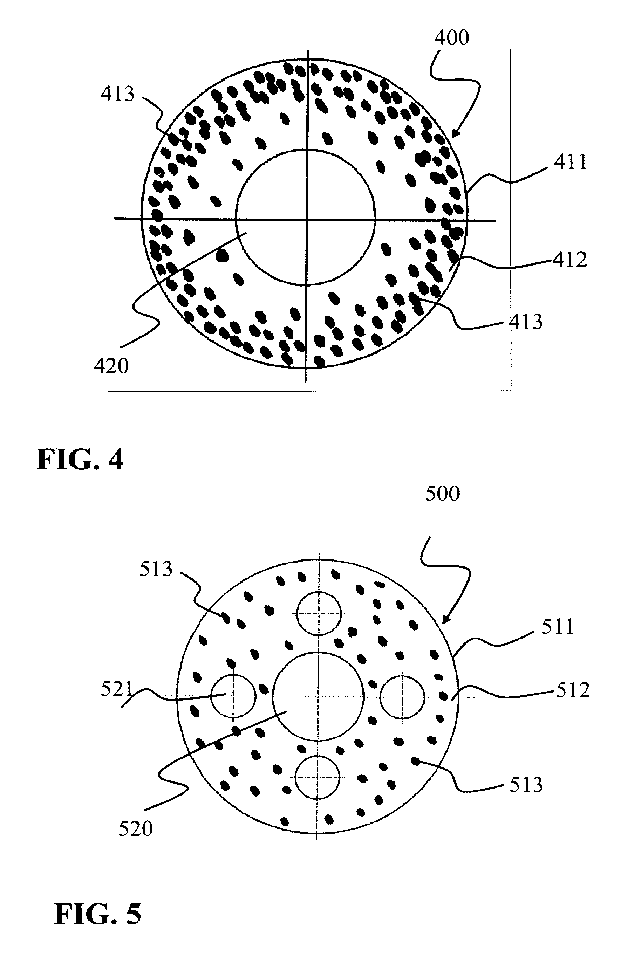

Der Aufbau des in Figur 4 dargestellten Isolationsschlauchs 400 entspricht im Wesentlichen dem in Figur 1 gezeigten Isolationsschlauch 100, wobei nun die Bariumtitanat-Partikel 413 ungleichmäßig über den Querschnitt verteilt sind. Die Konzentration der Bariumtitanat-Partikel 413 nimmt von innen nach außen zu. Dies hat den Vorteil, dass im äußeren Bereich des Querschnitts eine höhere Abriebfestigkeit erreicht wird, um den Isolationsschlauch 400 vor allem gegen äußere Reibung zu schützen.

In den in den Figuren 1 und 2 dargestellten Beispielen sind demgegenüber die abriebfesten Partikel 113, 313 in der jeweiligen Schicht 111, 311 gleichmäßig über den Querschnitt der jeweiligen Schicht 111, 311 verteilt.

Das in Figur 5 dargestellte vierte Beispiel eines Isolationsschlauchs 500 zeigt analog zu den in den Figuren 1 und 3 dargestellten Beispielen eine gleichmäßige Verteilung der Bariumtitanat-Partikel 513 über den Querschnitt der Schicht 511. Neben einer mittigen, durchgehenden, im Wesentlichen zylinderförmigen Öffnung 520 weist der Isolationsschlauch vier weitere durchgehende, im Wesentlichen zylinderförmige Öffnungen 521 auf, welche gleichmäßig um die mittige Öffnung 520 herum verteilt angeordnet sind. Die Mittelpunkte der Querschnitte der Öffnungen 521 sind etwa auf einem Kreis, jeweils etwa um 90° versetzt angeordnet. Der Querschnitt jeder Öffnung 521 hat einen kleineren Durchmesser als der Querschnitt der mittigen Öffnung 520. Die Öffnungen 520, 521 können im Einsatz für eine Elektrodenleitung wahlweise mit Wendel- oder Seilzuleitungen bestückt sein.

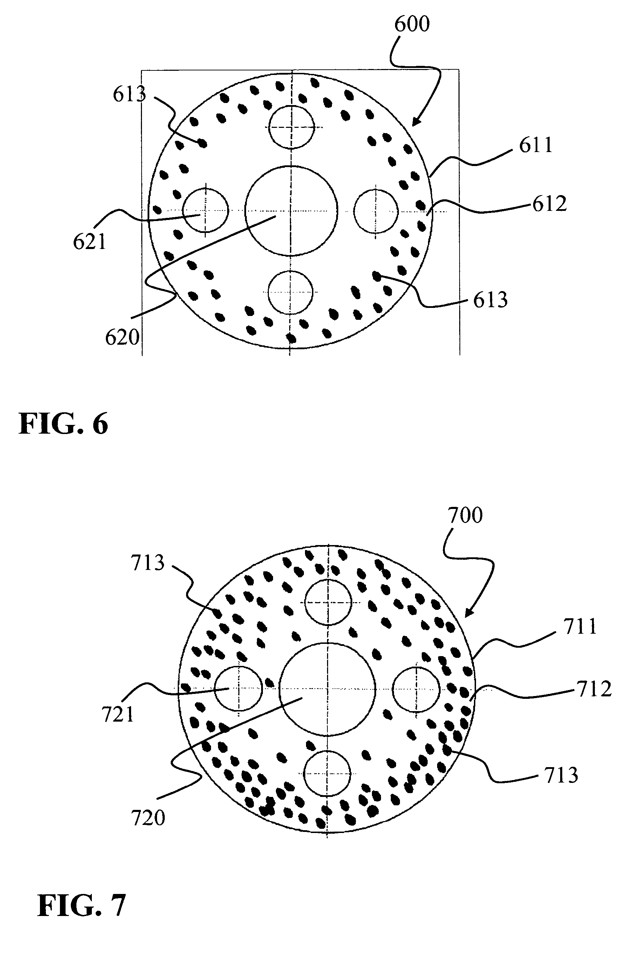

Das in Figur 7 dargestellte Beispiel eines Isolationsschlauchs 700 entspricht im Wesentlichen der in Figur 5 dargestellten Ausführungsform bis auf die Verteilung der Bariumtitanat-Partikel 713. Diese sind bei dem Isolationsschlauch 700 nicht gleichmäßig über den Querschnitt verteilt, sondern ihre Konzentration in der Schicht 711 nimmt von innen nach außen zu, d.h. die Konzentration der Partikel 713 weist einen Gradienten auf. In dem in Figur 6 dargestellten Beispiel eines Isolationsschlauchs 600 ist ebenfalls eine ungleichmäßige Verteilung der Bariumtitanat-Partikel 613 vorgesehen, wobei hier die Bariumtitanat-Partikel 613 im Wesentlichen nahe der äußeren Mantelfläche der Schicht 611 in einer äußeren Teilschicht angeordnet sind. Die Konzentration der Bariumtitanat-Partikel 613 steigt daher nicht graduell an, sondern eher sprunghaft in Richtung der äußeren Mantelfläche der Schicht 611.

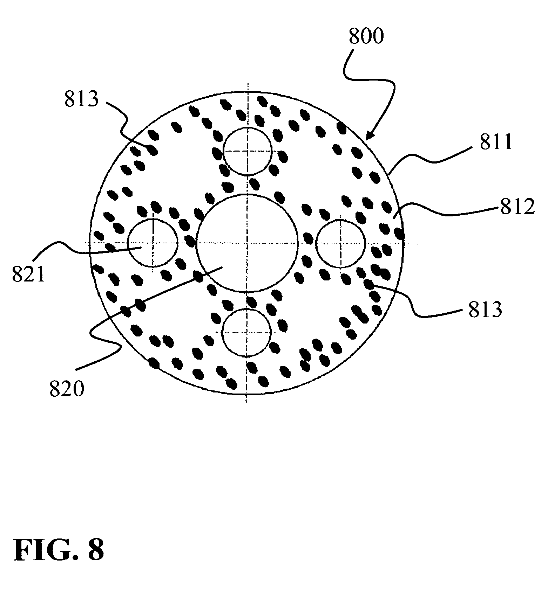

Auch bei dem in Figur 8 dargestellten Beispiel eines Isolationsschlauchs 800 sind die abriebfesten Bariumtitanat-Partikel 813 ungleichmäßig verteilt. Sie sind im Wesentlichen in Teilschichten der Schicht 811 zu finden, welche die äußere Mantelfläche und die durch die Öffnungen 820, 821 geformten Innenflächen ausbilden. Hierdurch wird insbesondere die Abriebfestigkeit gegenüber Reibepartnern, die in den Öffnungen 820, 821 angeordnet sind, erhöht. Auch bei diesem Beispiel steigt die Konzentration der Bariumtitanat-Partikel 813 in der Nähe der äußeren Mantelfläche und der Innenflächen der Öffnungen 820, 821 eher sprunghaft an.

Die Isolationsschläuche 400, 600, 700, 800 mit jeweils der ungleichmäßigen Verteilung der Partikel können beispielsweise durch Coextrusion hergestellt werden, nämlich indem zwei Materialien, von denen nur das eine mit Füllmaterial versetzt ist, gleichzeitig extrudiert werden. Alternativ können die ungleichmäßigen Verteilungen mittels Aufextruieren erzeugt werden, wobei die erste Schicht vorvernetzt ist. Die Variante, bei der das Füllmaterial an den Wandungen angeordnet ist (Isolationsschlauch 800), könnte durch Beimengung von Füllmaterial (insbesondere von Nanopartikeln) in den Luftstrom, der zum Aufrechthalten der Lumen erforderlich ist, erzielt werden. Gegebenenfalls kann der Effekt durch statische Aufladung der Partikel verbessert werden.

Ein Isolationsschlauch gemäß dem in Figur 1 abgebildeten Beispiel wurde mit verschiedenen Füllmaterialien mit erhöhter Abriebfestigkeit produziert, mittels eines Abriebtests untersucht und mit einem Isolationsschlauch, der kein Füllmaterial mit erhöhter Abriebfestigkeit enthielt, verglichen. Die unten dargestellte erste Tabelle zeigt die Ergebnisse für einen Abriebtest bei dem Abrieb an einer rauen Metalloberfläche, die auf dem Isolationsschlauch reibt. Bei dem Abriebtest wird der Schlauch mit einer definierten Kraft (hier z.B. 4 N) auf einen rotierenden Schleifkörper (hier z.B. Metall) mit definierter Rauigkeit, hier beispielsweise mit einer Rauigkeit von Rz 6,3, gedrückt. Der zurückgelegte Weg des Schleifkörpers (ausgedrückt in der Anzahl seiner Umdrehungen) im Verhältnis zum Abtrag des Materials ist ein Maß für die Abriebfestigkeit des Schlauches, welches bei identischen Schlauchgeometrien untereinander verglichen werden kann. Hierbei ist im Folgenden der Reibweg der zurückgelegte Weg des Schleifkörpers bei der definierten Kraft und der definierten Rauigkeit des Schleifkörpers, der benötigt wird, um einen Abtrag von 0,3 mm zu erreichen.

| Grundwerkstoff | Füllmaterial mit erhöhter Abriebfestigkeit | Anteil (in Vol.%) / Partikelgröße | Reibweg des metallischen Abriebpartners (Rauigkeit Rz 6,3 / Kraft 4 N) | Abriebresistenz (Faktor) |
|---|---|---|---|---|
| Silikonschlauch aus LSR, mit einer Härte von 50 Shore | - | 0% | 20 m | 1 |
| Silikonschlauch aus LSR, mit einer Härte von 50 Shore | Bariumtitanat | 15% / 3 µm | 60 m | 3 |
| Silikonschlauch aus LSR, mit einer Härte von 50 Shore | Tantal | 15% / 44 µm | 100 m | 5 |

Die oben dargestellten Ergebnisse zeigen, dass das Abriebverhalten eines Silikon-Isolationsschlauches mit dem angegebenen Füllmaterial gegenüber einem Silikon-Isolationsschlauch ohne Füllmaterial bei einem Abriebpartner aus Metall mit definierter Rauheit deutlich verbessert wird. Eine solche Verbesserung wird bereits durch eine mengenmäßig geringe Zugabe von lediglich 15 Vol% erreicht.

Zur Herstellung eines Isolationsschlauchs mit Elektrodenleitung gemäß der in den Figuren 1 bis 8 abgebildeten Beispiele, die jedoch nicht Teil der Erfindung sind, wird ein Grundwerkstoff extrudat mit dem Füllmaterial versetzt und sorgfältig vermischt, bevor es mit einem herkömmlichen Extruder für heißvernetzende Werkstoffe zu einem Schlauch extrudiert wird. In diesen Schlauch wird eine Mehrfachwendel als Elektrodenzuleitung eingeschoben und an den Enden mittels Adhesive (Klebstoff) fixiert.

### Bezugszeichenliste

- 4, 100, 300, 400, 500, 600, 700, 800: Isolationsschlauch
- 5: Schockelektrodenleitung
- 7: Elektrode
- 9: Ring-Elektrode
- 11: Tip-Elektrode
- 13: Y-Verteiler
- 15, 17: Steckerelement
- 111,311,411,511,611,711,811: Schicht
- 112, 312, 412, 512, 612, 712, 812: Grundwerkstoff
- 113, 313, 413, 513, 613, 713, 813: Partikel
- 120, 320, 420, 520, 620, 720, 820: Öffnung
- 521,621,721,821: Öffnung
- 330: Schicht
- L: Längsrichtung der Schockelektrodenleitung 5

## Patentansprüche

1. Isolationsschlauch (4, 100, 300, 400, 500, 600, 700, 800) für eine Elektrodenleitung (5) zur medizinischen Anwendung, insbesondere Herzschrittmacherelektrodenleitung, Defibrillatorelektrodenleitung, Elektrodenleitung zur Nervenstimulation oder dergl., enthaltend einen Grundwerkstoff (112, 312, 412, 512, 612, 712, 812) aus mindestens einem Material der Gruppe enthaltend Silikone, Polyurethane, Polyimid, PTFE, ETFE, Copolymere aus Silikonen und Polyurethanen, wobei der Isolationsschlauch (4, 100, 300, 400, 500, 600, 700, 800) zumindest in einem vorzugsweise die Mantelfläche des Isolationsschlauchs (4, 100, 300, 400, 500, 600, 700, 800) ausbildendem Volumenbereich zusätzlich zu dem Grundwerkstoff (112, 312, 412, 512, 612, 712, 812) ein faserförmiges Füllmaterial (113, 313, 413, 513, 613, 713, 813) aufweist, welches eine höhere Abriebfestigkeit als der jeweilige Grundwerkstoff (112, 312, 412, 512, 612, 712, 812) besitzt,
**dadurch gekennzeichnet, dass**
das faserförmige Füllmaterial mindestens überwiegend derart ausgerichtet ist, dass die Längsrichtung der Fasern parallel zu einer Längsachse des Isolationsschlauchs verläuft.

2. Isolationsschlauch (4, 100, 300, 400, 500, 600, 700, 800) nach Anspruch 1, **dadurch gekennzeichnet, dass** das Füllmaterial (113, 313, 413, 513, 613, 713, 813) mindestens ein Material der Gruppe enthaltend Kohlenstoff, Tantal, Platin, Gold, Iridium, Palladium, Silikonderivate, Copolymere aus Silikonen, keramisches Material, insbesondere Siliziumcarbid, Bariumtitanat, Titandioxid und Zirkoniumdioxid, und thermoplastisches Material, insbesondere Polyurethan, Polyamid, Polyethylen, Polyimid, PET, PFA, PTFE und ETFE, umfasst.

3. Isolationsschlauch (4, 100, 300, 400, 500, 600, 700, 800) nach einem der vorhergehenden Ansprüche, **dadurch gekennzeichnet, dass** der Anteil des Füllmaterials (113, 313, 413, 513, 613, 713, 813) an dem Material des Isolationsschlauchs (4, 100, 300, 400, 500, 600, 700, 800) 0,1 Vol.% bis 30 Vol.%, vorzugsweise 1 Vol.% bis 15 Vol.%, beträgt.

4. Isolationsschlauch (4, 100, 300, 400, 500, 600, 700, 800) nach einem der vorhergehenden Ansprüche, **dadurch gekennzeichnet, dass** die Länge des faserförmigen Füllmaterials 0,1 mm bis 10 mm beträgt.

5. Isolationsschlauch (4, 100, 300, 400, 500, 600, 700, 800) nach einem der vorhergehenden Ansprüche, **dadurch gekennzeichnet, dass** das Füllmaterial (313, 413, 613, 713, 813) über den Querschnitt des Isolationsschlauchs (300, 400, 600, 700, 800) ungleichmäßig verteilt angeordnet ist.

6. Isolationsschlauch (4, 100, 300, 400, 500, 600, 700, 800) nach einem der vorhergehenden Ansprüche, **dadurch gekennzeichnet, dass** der Volumenbereich zusätzlich zum faserförmigen Füllmaterial ein partikelförmiges Füllmaterial (113, 313, 413, 513, 613, 713, 813) aufweist.

7. Isolationsschlauch (4, 100, 300, 400, 500, 600, 700, 800) nach Anspruch 6, **dadurch gekennzeichnet, dass** das Füllmaterial (113, 313, 413, 513, 613, 713, 813) als kugel, bohnen- und/oder kornförmige Partikel und als Faser und/oder Faserabschnitt ausgebildet ist.

8. Isolationsschlauch (4, 100, 300, 400, 500, 600, 700, 800) nach Anspruch 6 oder 7, **dadurch gekennzeichnet, dass** der Durchmesser des partikelförmigen Füllmaterials (113, 313, 413, 513, 613, 713, 813) 1 nm bis 100 nm, vorzugsweise 20 nm bis 60 nm, beträgt.

9. Elektrodenleitung (5) zur medizinischen Anwendung, insbesondere Herzschrittmacherelektrodenleitung, Defibrillatorelektrodenleitung, Elektrodenleitung zur Nervenstimulation oder dergl., mit einem Isolationsschlauch (4, 100, 300, 400, 500, 600, 700, 800) nach einem der vorhergehenden Ansprüche, wobei der Isolationsschlauch (4, 100, 300, 400, 500, 600, 700, 800) in dem Abschnitt der Elektrodenleitung (5), in dem er entlang dieser angeordnet ist, ein Leiterelement außen umgibt.

## Claims

1. An insulating tube (4, 100, 300, 400, 500, 600, 700, 800) for an electrode lead (5) for medical use, in particular a pacemaker electrode lead, a defibrillator electrode lead, an electrode lead for nerve stimulation or the like, containing a base material (112, 312, 412, 512, 612, 712, 812) made of at least one material from the group comprising silicones, polyurethanes, polyimide, PTFE, ETFE, copolymers made of silicones and polyurethanes, wherein the insulating tube (4, 100, 300, 400, 500, 600, 700, 800) comprises a fibrous filler material (113, 313, 413, 513, 613, 713, 813) having a higher abrasion resistance than the base material (112, 312, 12, 512, 612, 712, 812) at least in a volume region that preferably forms the lateral surface of the insulating tube (4, 100, 300, 400, 500, 600, 700, 800) in addition to the base material (112, 312, 412, 512, 612, 712, 812),
**characterized in that**
the fibrous filler material is at least predominantly oriented such that the longitudinal direction of the fibers extends parallel to a longitudinal axis of the insulating tube.

2. The insulating tube (4, 100, 300, 400, 500, 600, 700, 800) according to claim 1, **characterized in that** the filler material (113, 313, 413, 513, 613, 713, 813) comprises at least one material from the group containing carbon, tantalum, platinum, gold, iridium, palladium, silicone derivatives, copolymers of silicones, ceramic material, in particular silicon carbide, barium titanate, titanium dioxide and zirconium dioxide, and thermoplastic material, in particular polyurethane, polyamide, polyethylene, polyimide, PET, PFA, PTFE and ETFE.

3. The insulating tube (4, 100, 300, 400, 500, 600, 700, 800) according to any one of the preceding claims, **characterized in that** the portion of the filler material (113, 313, 413, 513, 613, 713, 813) in the material of the insulating tube (4, 100, 300, 400, 500, 600, 700, 800) is 0.1 vol.% to 30 bol.%, preferably 1 vol.% to 15 vol.%.

4. The insulating tube (4, 100, 300, 400, 500, 600, 700, 800) according to any one of the preceding claims, **characterized in that** the length of the fibrous filler material is 0.1 mm to 10 mm.

5. The insulating tube (4, 100, 300, 400, 500, 600, 700, 800) according to any one of the preceding claims, **characterized in that** the filler material (313, 413, 613, 713, 813) is distributed unevenly over the cross-section of the insulating tube (300, 400, 600, 700, 800).

6. The insulating tube (4, 100, 300, 400, 500, 600, 700, 800) according to any one of the preceding claims, **characterized in that** the volume region contains a particulate filler material (113, 313, 413, 513, 613, 713, 813) in addition to the fibrous filler material.

7. The insulating tube (4, 100, 300, 400, 500, 600, 700, 800) according to claim 6, **characterized in that** the filler material (113, 313, 413, 513, 613, 713, 813) is configured as spherical, bean-shaped, and/or grain-shaped particles and as fibers and/or fiber sections.

8. The insulating tube (4, 100, 300, 400, 500, 600, 700, 800) according to claim 6 or 7, **characterized in that** the diameter of the particulate filler material (113, 313, 413, 513, 613, 713, 813) is 1 nm to 100 nm, preferably 20 nm to 60 nm.

9. An electrode lead (5) for medical use, in particular a pacemaker electrode lead, a defibrillator electrode lead, an electrode lead for nerve stimulation or the like, comprising an insulating tube (4, 100, 300, 400, 500, 600, 700, 800) according to any one of the preceding claims, wherein the insulating tube (4, 100, 300, 400, 500, 600, 700, 800) externally surrounds a lead element in the section of the electrode lead (5) in which said insulating tube is disposed along said electrode lead.

## Revendications

1. Tube d'isolation (4, 100, 300, 400, 500, 600, 700, 800) pour une ligne d'électrodes (5) pour une application médicale, en particulier une ligne d'électrodes de stimulateur cardiaque, une ligne d'électrodes de défibrillateur, une ligne d'électrodes pour la stimulation nerveuse ou autres, contenant un matériau de base (112, 312, 412, 512, 612, 712, 812) issu d'au moins un matériau du groupe comprenant des silicones, du polyuréthane, du polyimide, du PTFE, de l'ETFE, des copolymères de silicones et de polyuréthanes, le tube d'isolation (4, 100, 300, 400, 500, 600, 700, 800) comportant, en plus du matériau de base (112, 312, 412, 512, 612, 712, 812), un matériau de remplissage fibreux (113, 313, 413, 513, 613, 713, 813) présentant une résistance au frottement supérieure à celle du matériau de base (112, 312, 412, 512, 612, 712, 812) respectif, au moins dans une région volumique formant de préférence la surface extérieure du tube d'isolation (4, 100, 300, 400, 500, 600, 700, 800),
**caractérisé en ce que**
le matériau de remplissage fibreux est au moins majoritairement orienté de manière à ce que le sens longitudinal des fibres s'étende parallèlement à un axe longitudinal du tube d'isolation.

2. Tube d'isolation (4, 100, 300, 400, 500, 600, 700, 800) selon la revendication 1, **caractérisé en ce que** le matériau de remplissage (113, 313, 413, 513, 613, 713, 813) comprend au moins un matériau du groupe contenant du carbone, du tantale, du platine, de l'or, de l'iridium, du palladium, des dérivés de silicone, des copolymères de silicone, du matériau céramique, en particulier du carbure de silicium, du titanate de baryum, du dioxyde de titane et du dioxyde de zirconium, et du matériau thermoplastique, en particulier du polyuréthane, du polyamide, du polyéthylène, du polyimide, du PET, du PFA, du PTFE et de l'ETFE.

3. Tube d'isolation (4, 100, 300, 400, 500, 600, 700, 800) selon l'une des revendications précédentes, **caractérisé en ce que** la part du matériau de remplissage (113, 313, 413, 513, 613, 713, 813) dans le matériau du tube d'isolation (4, 100, 300, 400, 500, 600, 700, 800) est de 0,1% vol. à 30% vol., de préférence de 1% vol. à 15% vol.

4. Tube d'isolation (4, 100, 300, 400, 500, 600, 700, 800) selon l'une des revendications précédentes, **caractérisé en ce que** la longueur du matériau de remplissage fibreux mesure entre 0,1 mm et 10 mm.

5. Tube d'isolation (4, 100, 300, 400, 500, 600, 700, 800) selon l'une des revendications précédentes, **caractérisé en ce que** le matériau de remplissage (313, 413, 513, 613, 713, 813) est réparti de façon in homogène sur la section transversale du tube d'isolation (300, 400, 500, 600, 700, 800).

6. Tube d'isolation (4, 100, 300, 400, 500, 600, 700, 800) selon l'une des revendications précédentes, **caractérisé en ce que** la région volumique comporte un matériau de remplissage sous forme de particules (113, 313, 413, 513, 613, 713, 813) en plus du matériau de remplissage fibreux.

7. Tube d'isolation (4, 100, 300, 400, 500, 600, 700, 800) selon la revendication 6, **caractérisé en ce que** le matériau de remplissage (113, 313, 413, 513, 613, 713, 813) se présente comme des particules sphériques, des particules en forme de fèves et/ou de granules et comme des fibres et/ou des sections de fibres.

8. Tube d'isolation (4, 100, 300, 400, 500, 600, 700, 800) selon la revendication 6 ou 7, **caractérisé en ce que** le diamètre du matériau de remplissage sous forme de particules (113, 313, 413, 513, 613, 713, 813) mesure de 1nm à 100 nm, de préférence de 20 nm à 60 nm.

9. Ligne d'électrodes (5) pour une application médicale, en particulier une ligne d'électrodes de stimulateur cardiaque, une ligne d'électrodes de défibrillateur, une ligne d'électrodes pour la stimulation nerveuse ou autres, avec un tube d'isolation (4, 100, 300, 400, 500, 600, 700, 800) selon l'une des revendications précédentes, le tube d'isolation (4, 100, 300, 400, 500, 600, 700, 800) entourant un élément conducteur dans la section de la ligne d'électrodes (5) dans laquelle il est agencé le long de celle-ci.
